# EUROPEAN PATENT APPLICATION

(11) **EP 4 183 358 A1**
(43) Date of publication of application: **24.05.2023**
(21) Application number: 22207989.9
(22) Date of filing: 17.11.2022
(51) Int. Cl.: A61B 18/14

(54) **JAW TIP SUPPORT FOR TAPERED JAW MEMBER**

(30) Priority: 19.11.2021 US 202163281185 P; 03.10.2022 US 202217958692
(71) Applicant: Covidien LP, Mansfield, MA 02048 (US)
(72) Inventor: SIEBENALLER, Curtis M., Boulder, 80301 (US); FRY, Monte S., Boulder, 80301 (US); KINGSLEY, Dylan R., Boulder, 80301 (US)
(74) Representative: Maschio & Soames IP Ltd

(57) **Abstract**

A jaw member of an electrosurgical instrument includes a U-shaped structural frame defining a cavity therein and extending therealong configured to receive at least a portion of an insulative spacer therein such that the insulative spacer extends distally therefrom. The insulative spacer includes a tapered fin at a distal end thereof. A tissue treating plate is disposed on the face of the insulative spacer, the tissue treating plate is adapted to connect to a source of energy to treat tissue therewith. The tapered fin is configured to extend to and support a distal end of the tissue treating plate.

## Description

### CROSS REFERENCE TO RELATED APPLICATION

This application claims the benefit of U.S. Provisional Application Serial No. 63/281,185 filed November 19, 2021, the entire contents of which being incorporated by reference herein.

### FIELD

The present disclosure relates to surgical instruments and, more specifically, to end effector assemblies for surgical instruments, such as for use in robotic surgical systems.

### BACKGROUND

Robotic surgical systems are increasingly utilized in various different surgical procedures. Some robotic surgical systems include a console supporting a robotic arm. One or more different surgical instruments may be configured for use with the robotic surgical system and selectively mountable to the robotic arm. The robotic arm provides one or more inputs to the mounted surgical instrument to enable operation of the mounted surgical instrument.

A surgical forceps, one type of instrument capable of being utilized with a robotic surgical system, relies on mechanical action between its jaw members to grasp, clamp, and constrict tissue. Electrosurgical forceps utilize both mechanical clamping action and energy to heat tissue to treat, e.g., coagulate, cauterize, or seal, tissue. Typically, once tissue is treated, the tissue is severed using a cutting element. Certain electrosurgical forceps utilize a tapered jaw design to facilitate dissection and access to smaller operating cavities. Manufacturing a jaw member with a consistent strength and stiffness profiles to absorb the necessary closure pressures associated with tissue sealing can present design challenges especially at the tapered distal tip.

### SUMMARY

As used herein, the term "distal" refers to the portion that is being described which is further from an operator (whether a human surgeon or a surgical robot), while the term "proximal" refers to the portion that is being described which is closer to the operator. The terms "about," substantially," and the like, as utilized herein, are meant to account for manufacturing, material, environmental, use, and/or measurement tolerances and variations, and in any event may encompass differences of up to 10%. Further, to the extent consistent, any of the aspects described herein may be used in conjunction with any or all of the other aspects described herein.

Provided in accordance with aspects of the present disclosure is a jaw member of an electrosurgical instrument which includes a U-shaped structural frame defining a cavity therein and extending therealong configured to receive at least a portion of an insulative spacer therein such that the insulative spacer extends distally therefrom. The insulative spacer includes a tapered fin at a distal end thereof. A tissue treating plate is disposed on the face of the insulative spacer, the tissue treating plate is adapted to connect to a source of energy to treat tissue therewith. The tapered fin is configured to extend to and support a distal end of the tissue treating plate.

In an aspect of the present disclosure, the insulative spacer is configured to electrically isolate the structural frame from the tissue treating plate.

In an aspect of the present disclosure, the tapered fin includes an overhang on either side thereof configured to laterally support the distal end of the tissue treating plate.

In an aspect of the present disclosure, the jaw member includes an outer insulative jacket disposed about at least a portion of an outer face of the structural frame and the insulative spacer. In other aspects of the present disclosure, the outer insulative jacket is overmolded about the structural frame and the insulative spacer.

In an aspect of the present disclosure, the insulative spacer is made from a material selected from the group consisting of: PPA, PTFE, PEEK, PBI and PEI.

In an aspect of the present disclosure, the draft angle of the tapered fin is about 0.5 degrees. The draft angle may be in the range of about 3 degrees to about 10 degrees.

In an aspect of the present disclosure, the insulative spacer is configured to receive a thermal cutting element therein and extending therealong. In other aspects of the present disclosure, the insulative spacer is configured to electrically isolate the structural frame from the thermal cutting element.

Provided in accordance with aspects of the present disclosure is an end effector of an electrosurgical instrument which includes first and second jaw members pivotably coupled to one another such that at least one of the first or second jaw members is movable relative to the other from a spaced-apart position to an approximated position to grasp tissue therebetween. Each of the first and/or second jaw members includes: a U-shaped structural frame defining a cavity therein and extending therealong configured to receive at least a portion of an insulative spacer therein such that the insulative spacer extends distally therefrom, the insulative spacer includes a tapered fin at a distal end thereof; and a tissue treating plate disposed on the face of the insulative spacer, the tissue treating plates of each jaw member disposed in vertical opposition relative to one another and each tissue treating plate is adapted to connect to an opposite potential from a source of energy to treat tissue therewith, each tapered fin is configured to extend to and support a distal end of the respective tissue treating plate.

In an aspect of the present disclosure, each insulative spacer is configured to electrically isolate the structural frame from the tissue treating plate of each respective jaw member.

In an aspect of the present disclosure, each tapered fin includes an overhang on either side thereof configured to laterally support the distal end of the tissue treating plate of each respective jaw member.

In an aspect of the present disclosure, each jaw member includes an outer insulative jacket disposed about at least a portion of an outer face of the structural frame and the insulative spacer of each respective jaw member. In other aspects of the present disclosure, the outer insulative jacket is overmolded about the structural frame and the insulative spacer of each respective jaw member.

In an aspect of the present disclosure, the insulative spacer of each jaw member is made from a material selected from the group consisting of: PPA, PTFE, PEEK, PBI and PEI.

In an aspect of the present disclosure, the draft angle of each tapered fin is about 0.5 degrees. The draft angle may be in the range of about 3 degrees to about 10 degrees.

In an aspect of the present disclosure, the insulative spacer of the first jaw member is configured to receive a thermal cutting element therein and extending therealong. In other aspects of the present disclosure, the insulative spacer of the first jaw member is configured to electrically isolate the structural frame from the thermal cutting element.

### BRIEF DESCRIPTION OF THE DRAWINGS

Various aspects and features of the present disclosure are described hereinbelow with reference to the drawings wherein:
FIG. 1 is a perspective view of a surgical instrument in accordance with the present disclosure configured for mounting on a robotic arm of a robotic surgical system;
FIG. 2 is a rear perspective view of a proximal portion of the surgical instrument of FIG. 1 with an outer housing removed;
FIG. 3 is a schematic illustration of an exemplary robotic surgical system configured to releasably receive the surgical instrument of FIG. 1.
FIG. 4 is a side view of an end effector assembly of the forceps of FIG. 1 including first and second jaw members;
FIG. 5 is a perspective view of a first jaw member of the end effector assembly with outer insulative jackets removed therefrom to expose an insulative spacer;
FIG. 6 is a transverse, cross-sectional view of the first jaw member of the end effector assembly as shown in FIG. 4; and
FIG. 7 is an enlarged view of the distal tapered fin of the insulative spacer of the jaw member of FIG. 5.

### DETAILED DESCRIPTION

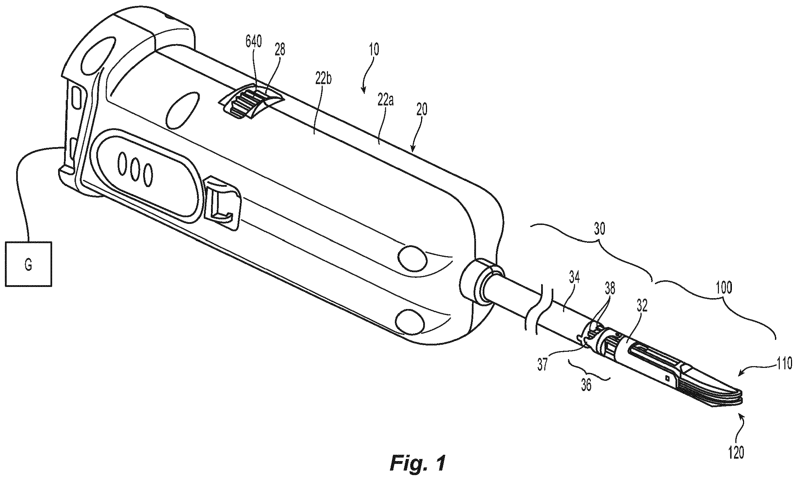

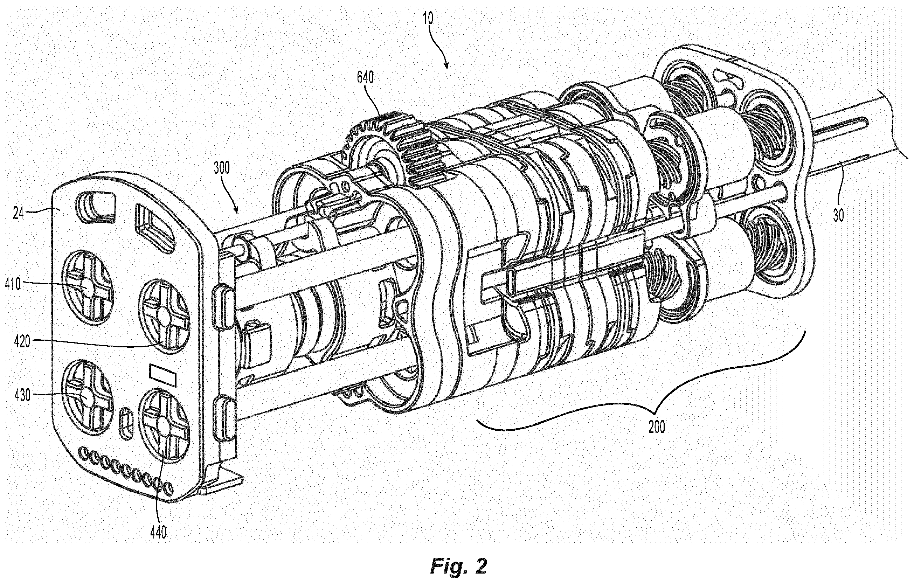

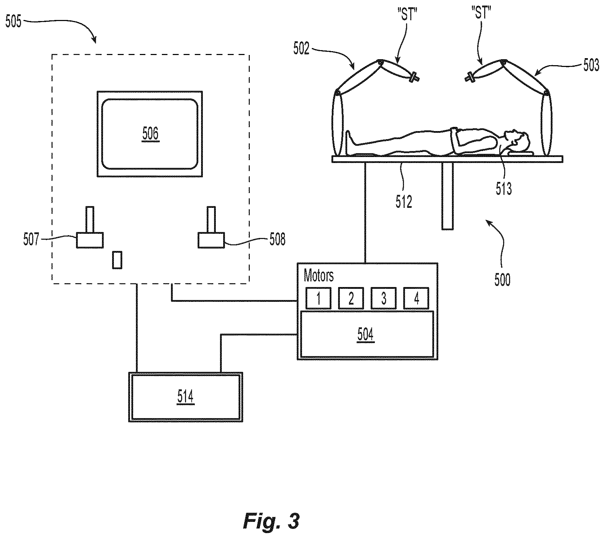

Referring to FIGS. 1 and 2, a surgical instrument 10 provided in accordance with the present disclosure generally includes a housing 20, a shaft 30 extending distally from the housing 20, an end effector assembly 100 extending distally from the shaft 30, and an actuation assembly 300 disposed within the housing 20 and operably associated with the shaft 30 and the end effector assembly 100. The surgical instrument 10 is detailed herein as an articulating electrosurgical forceps configured for use with a robotic surgical system, e.g., robotic surgical system 500 (FIG. 3). However, the aspects and features of the surgical instrument 10 provided in accordance with the present disclosure, detailed below, are equally applicable for use with other suitable surgical instruments (including non-robotic surgical instrument) and/or in other suitable surgical systems (including non-robotic surgical systems).

The housing 20 of the surgical instrument 10 includes first and second body portions 22a, 22b and a proximal faceplate 24 (FIG. 2) that cooperate to enclose the actuation assembly 300 therein. The proximal faceplate 24 includes apertures defined therein through which inputs 410, 420, 430, 440 of the actuation assembly 300 extend. A pair of latch levers extends outwardly from opposing sides of the housing 20 and enables releasable engagement (directly or indirectly) of the housing 20 with a robotic arm of a surgical system, e.g., robotic surgical system 500 (FIG. 3). An aperture 28 defined through the housing 20 permits a thumbwheel 640 to extend therethrough to enable manual manipulation of the thumbwheel 640 from the exterior of the housing 20 to permit manual opening and closing of the end effector assembly 100.

The shaft 30 of the surgical instrument 10 includes a distal segment 32 (such as, for example, a collar or clevis), a proximal segment 34, and an articulating section 36 disposed between the distal and proximal segments 32, 34, respectively. The articulating section 36 includes one or more articulating components 37, e.g., links, joints, etc. A plurality of articulation cables 38, e.g., four (4) articulation cables, or other suitable actuators, extends through the articulating section 36. More specifically, the articulation cables 38 are operably coupled to the distal segment 32 of the shaft 30 at the distal ends thereof and extend proximally from the distal segment 32 of the shaft 30, through the articulating section 36 and the proximal segment 34 of the shaft 30, and into the housing 20, wherein the articulation cables 38 operably couple with an articulation assembly 200 of the actuation assembly 300 to enable selective articulation of the distal segment 32 (and, thus the end effector assembly 100) relative to the proximal segment 34 and the housing 20, e.g., about at least two axes of articulation (yaw and pitch articulation, for example). The articulation assembly 200 is operably coupled between the first and second inputs 410, 420, respectively, of the actuation assembly 300 and the articulation cables 38 (FIG. 1) such that, upon receipt of appropriate rotational inputs into the first and/or second inputs 410, 420, the articulation assembly 200 manipulates the articulation cables 38 to articulate the end effector assembly 100 in a desired direction, e.g., to pitch and/or yaw the end effector assembly 100. The articulation cables 38 are arranged in a generally rectangular configuration, although other suitable configurations are also contemplated.

With respect to articulation of the end effector assembly 100 relative to the proximal segment 34 of the shaft 30, actuation of the articulation cables 38 is effected in pairs. More specifically, in order to pitch the end effector assembly 100, the upper pair of cables 38 is actuated in a similar manner while the lower pair of cables 38 is actuated in a similar manner relative to one another but an opposite manner relative to the upper pair of cables 38. With respect to yaw articulation, the right pair of cables 38 is actuated in a similar manner while the left pair of cables 38 is actuated in a similar manner relative to one another but an opposite manner relative to the right pair of cables 38.

Turning now to FIG. 3, a robotic surgical system 500 is configured for use in accordance with the present disclosure. Aspects and features of the robotic surgical system 500 not germane to the understanding of the present disclosure are omitted to avoid obscuring the aspects and features of the present disclosure in unnecessary detail.

The robotic surgical system 500 generally includes a plurality of robot arms 502, 503; a control device 504; and an operating console 505 coupled with control device 504. The operating console 505 may include a display device 506, which may be set up in particular to display three-dimensional images; and manual input devices 507, 508, by means of which a person, e.g., a surgeon, may be able to telemanipulate the robot arms 502, 503 in a first operating mode. The robotic surgical system 500 may be configured for use on a patient 513 lying on a patient table 512 to be treated in a minimally invasive manner. The robotic surgical system 500 may further include a database 514, in particular coupled to the control device 504, in which are stored, for example, pre-operative data from the patient 513 and/or anatomical atlases.

Each of the robot arms 502, 503 may include a plurality of members, which are connected through joints, and a mounted device which may be, for example, a surgical tool "ST." One or more of the surgical tools "ST" may be the surgical instrument 10 (FIG. 1), thus providing such functionality on a robotic surgical system 500.

Specifically, the actuation assembly 300 (FIG. 2) is configured to operably interface with the robotic surgical system 500 when the surgical instrument 10 is mounted on the robotic surgical system 500 to enable robotic operation of the actuation assembly 300. That is, the robotic surgical system 500 selectively provides rotational inputs to inputs 410, 420, 430, 440 of the actuation assembly 300 to articulate the end effector assembly 100, grasp tissue between the first and second jaw members 110, 120, and/or cut tissue grasped between the first and second jaw members 110, 120.

The robot arms 502, 503 may be driven by electric drives, e.g., motors, connected to the control device 504. The control device 504, e.g., a computer, may be configured to activate the motors, in particular by means of a computer program, in such a way that the robot arms 502, 503, and, thus, their mounted surgical tools "ST" execute a desired movement and/or function according to a corresponding input from the manual input devices 507, 508, respectively. The control device 504 may also be configured in such a way that it regulates the movement of the robot arms 502, 503 and/or of the motors.

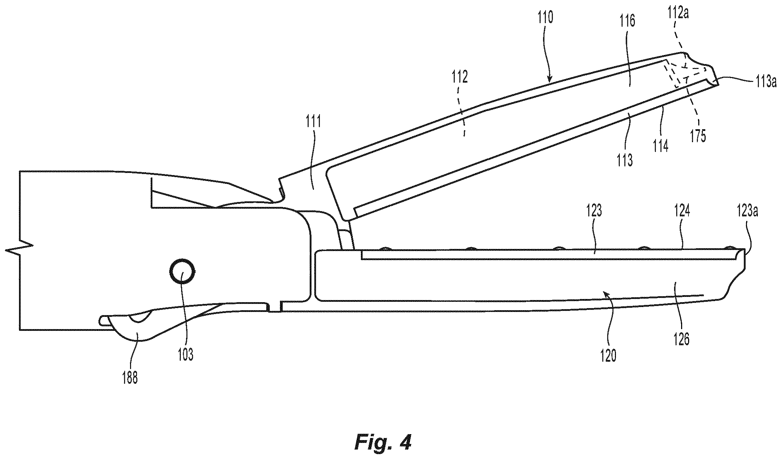

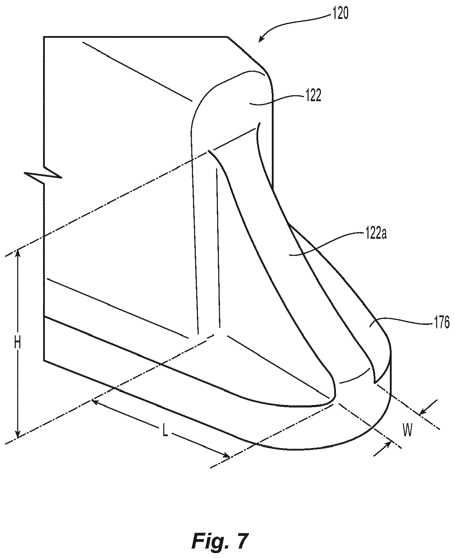

Turning to FIGS. 4-7, end effector assembly 100, as noted above, includes first and second jaw members 110, 120. Either or both jaw members 110, 120 may include a structural frame 111, 121, an insulative spacer 112, 122, a tissue treating plate 113, 123 defining the respective tissue treating surface 114, 124 thereof, and, in aspects, an outer insulative jacket 116, 126. Tissue treating plates 113, 123 may be pre-formed and engaged with insulative spacers 112, 122 and/or other portion(s) of jaw members 110, 120 via, for example, overmolding, adhesion, mechanical engagement, etc., or may be deposited onto insulative spacers 112, 122, e.g., via sputtering or other suitable deposition technique.

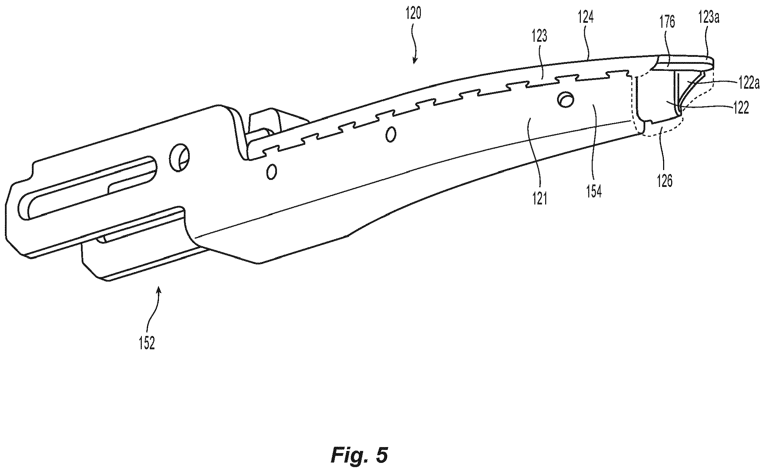

Referring in particular to FIG. 5, jaw member 120, as noted above, includes a generally U-shaped structural frame 121, an insulative spacer 122, a tissue treating plate 123 defining a tissue treating surface 124, and, in aspects, an outer insulative jacket 126 (FIG. 4). Structural frame 121 may be formed from stainless steel or other suitable material configured to provide structural support to jaw member 120. Structural frame is typically stamped in a U-shaped configuration and defines a cavity 121a defined therealong configured to at least partially receive the insulative space 122. The frame may be forged, cast or otherwise fabricated. Structural frame 121 includes a proximal flange portion 152 about which jaw member 120 is pivotably coupled to jaw member 110 via pivot 103 (FIG. 4) and a distal body portion 154 that supports the other components of jaw member 120, e.g., insulative spacer 122, tissue treating plate 123, and outer insulative jacket 126 (where provided). In shaft-based or robotic configurations, proximal flange portion 152 enables operable coupling of jaw member 120 to the actuation assembly 200 to enable pivoting of jaw member 110 relative to jaw member 120 in response to actuation thereof. Many suitable drive arrangements are contemplated, e.g., using cam pins and cam slots, a screw-drive mechanism, etc.

Insulative spacer 122 of jaw member 120 is formed from an electrically insulative material capable of withstanding high temperatures, e.g., above at least 300°C, although other configurations are also contemplated. Insulative spacer 122 may be formed from ceramic or other suitable material, e.g., PPA, PTFE, PEEK, PBI and PEI. Insulative spacer 122 may include various cutout or apertures defined therealong to facilitate assembly of jaw member 120.

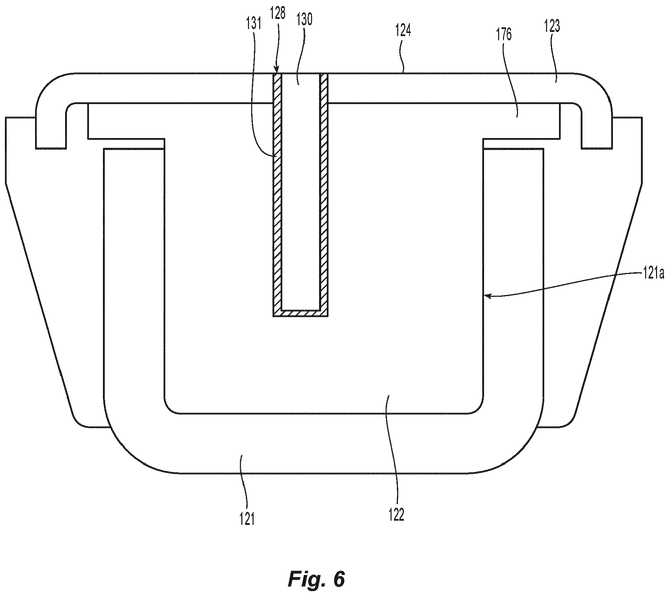

Continuing with reference to FIG. 5, insulative spacer 122 is configured for at least partial receipt within distal body portion 154 of structural frame 121 and may be at least partially shaped complementary thereto. Insulative spacer 122 may include one or more alignment features to facilitate assembly to frame 121, e.g., alignment bosses, flanges, etc. Overhang 176 of insulative spacer 122 is configured to be supported on longitudinally extending sides of the distal body portion 154 of structural frame 121 (FIG. 6). Overhang 176 may also be configured to provide electrical insulation between the seal plate and other structural components.

Referring again to FIGS. 4-7, as noted above, tissue treating plate 123 is supported or received on insulative spacer 122. In aspects, tissue treating plate 123 overlaps the overhangs 176 of insulative spacer 122, although other configurations are also contemplated. As noted above, tissue treating plate 123 may be pre-formed and engaged with insulative spacer 122 or may be deposited onto insulative spacers 122, e.g., via sputtering or other suitable deposition technique. In aspects where tissue treating plate 123 is pre-formed and engaged with insulative spacer 122, tissue treating plate 123 may be secured to jaw member 120 and, thus, insulative spacer 122, via overmolding of outer insulative jacket 126 about distal body portion 154 of structural frame 121 of tissue treating plate 123. Tissue treating plate 123 may include a longitudinally extending slot 128 defined therethrough and along at least a portion of the length thereof.

Slot 128 may be transversely centered on tissue treating surface 124 or may be offset relative thereto and may be linear, curved, include angled sections, etc. similarly or differently from the configuration, e.g., curvature, of jaw member 120. Slot 128 exposes a portion of insulative spacer 122 and may be recessed relative to tissue treating surface 124, substantially co-planar with tissue treating surface 124, or protruding beyond tissue treating surface 124 towards jaw member 110. In other aspects, slot 128 is omitted and, thus tissue treating plate 123 extends continuously insulative spacer 122 without exposing any portion thereof.

Regardless of the particular configuration of tissue treating plate 123, insulative spacer 122 electrically isolates tissue treating plate 123 from structural frame 121. Tissue treating plate 123 is electrically connected, e.g., via one or more electrical leads (not shown), to an activation switch (not shown)) and an electrosurgical generator "G" (FIG. 1) to enable selective energization of tissue treating plate 123, e.g., as one pole of a bipolar Radio Frequency (RF) electrosurgical circuit. However, other suitable energy modalities, e.g., thermal, ultrasonic, light, microwave, infrared, etc., are also contemplated. Jaw member 120 further includes a thermal cutting element 130 which may be connected to the same (e.g., generator "G") or alternate electrical energy source. A mechanical cutting element (not shown) may also be employed which is configured to translate a cutting blade through the tissue upon selective application thereof.

A second activation switch (not shown) may be integrated with the robotic system 500 and coupled to the thermal cutting element 130 and to the electrosurgical generator "G" for enabling the selective activation of the supply of energy to the thermal cutting element 130 for thermally cutting tissue.

Thermal cutting element 130 may be secured within and directly to insulative spacer 122 in any suitable manner, e.g., adhesive, friction fitting, mechanical engagement, etc., or may be indirectly secured within insulative spacer 122 via attachment to one or more other components of jaw member 120. Thermal cutting element 130 may protrude distally beyond the distal tip of insulative spacer 122 of jaw member 120, may be substantially flush therewith, or may be recessed relative thereto. In aspects where end effector assembly 100, or a portion thereof, is curved, thermal cutting element 130 may similarly be curved. Thermal cutting element 130 is electrically connected, e.g., via one or more electrical leads (not shown), to electrosurgical generator "G" (FIG. 1) to enable selective activation of the supply of energy to thermal cutting element 130 for heating thermal cutting element 130 to thermally cut tissue. Thermal cutting element 130, more specifically, may be configured to cut previously (or concurrently) sealed tissue grasped between jaw members 110, 120, to cut tissue extending across jaw member 120, and/or to cut tissue adjacent the distal end of jaw member 120.

Thermal cutting element 130 may be any suitable thermal cutting element such as, for example, a resistive cutting element, a ferromagnetic cutting element, a monopolar cutting element, a bipolar cutting element, etc. With respect to resistive cutting elements, thermal cutting element 130 may include a substrate, e.g., aluminum, ceramic, stainless steel, etc., an insulative coating disposed on the substrate, e.g., a Plasma Electrolytic Oxidation (PEO)-formed coating, a sprayed coating, a deposited coating, or other suitable coating, and a heating circuit trace disposed on the coating such that when an AC voltage is applied to the heating circuit trace, the thermal cutting element 130 is heated for thermally cutting tissue in contact therewith or adjacent thereto.

With reference to FIG. 4, jaw member 110 includes a structural frame 111, an insulative spacer 112, a tissue treating plate 113 defining tissue treating surface 114, and, in aspects, an outer insulative jacket 116. Jaw member 110 is similar to jaw member 120 with the general exception of thermal cutting element 130 being housed therein.

Structural frame 121 of jaw member 120 defines a proximal flange portion 152 and a distal body portion 154 extending distally from proximal flange portion 152. Proximal flange portion 152 may be bifurcated to define a pair of spaced apart proximal flange portion segments or may define any other suitable configuration. Proximal flange portion 152 of jaw member 120 and proximal flange portion 188 of jaw member 110 may define a nestled configuration, e.g., wherein one of the proximal flange portions 152, 188 is received within the other, an overlapping configuration, e.g., wherein proximal flange portions 152, 188 at least partially overlap one another, or an offset configuration, e.g., wherein proximal flange portions 152, 188 are positioned in side-by-side relation. Regardless of the particular arrangement of proximal flange portions 152, 188, proximal flanges are configured for receipt of pivot 103, e.g., welded or otherwise secured therein, to pivotably couple jaw members 110, 120 with one another.

Insulative spacer 112 of jaw member 110 may be configured similarly as and may include any of the features of insulative spacer 122 of jaw member 120 and, thus, only differences therebetween are described below. For example, tissue treating plate 113 defines tissue treating surface 114 and is supported on insulative spacer 112 similarly as tissue treating plate 123 is supported on insulative spacer 122. Tissue treating plate 113 may be formed similarly to and/or include any of the features of tissue treating plate 123 and may be secured to jaw member 110 similarly as tissue treating plate 123 is secured to jaw member 120, e.g., via overmolding of outer insulative jacket 116. Insulative spacer 112 electrically isolates tissue treating plate 113 from structural frame 111.

Details relating to the spacer 122 are described in more detail below with reference to FIGS. 4-7. More particularly, spacer 122 includes a reduced profile at a distal end thereof that is shaped like a fin 122a that extends under and supports the corresponding distal end 123a of the tissue treating plate 123. In order to reduce the configuration of the jaw members 110, 120 (only jaw member 120 is shown in detail but the configuration of the spacer 112 is substantially the same) to a tapered profile at a distal end thereof, the stamped (or otherwise fabricated), U-shaped configuration of the structure frame, e.g., frame 121, is reduced in length proximate the distal end of the jaw member 120 for manufacturing purposes. In other words, the thinner the profile of the frame 121 the more difficult it is to form the U-shape at a distal end. As a result, the structural frame, e.g., frame 121, does not support the distal end 123a of the tissue treating plate 123.

To avoid inconsistencies in pressure between the jaw members 110, 120 along the length thereof during sealing, the distal ends 113a, 123a of the of the jaw members 110, 120, respectively, are supported by the corresponding fins 112a (FIG. 4), 122a associated with each of the same. Overhangs 175 (FIG. 4) and 176 (FIG. 6), which extend laterally from respective fins 112a, 122a, transversally support the distal tips 113a, 123a of the tissue treating plates 113, 123 to insure sealing pressure is generated along and across and the same. This support geometry also allows the tissue treating plates 113, 123 to "fully seat" near the jaw tip, thus reducing the opportunity for flash to leak around and over the distal end of the tissue treating plates 113, 123 during molding.

Fins 112a, 122a are made from the same materials as the spacers 112, 122 mentioned above, namely, ceramic or other suitable material, e.g., PPA, Polytetrafluoroethylene (PTFE), polyetheretherketone (PEEK), Polyetherimide (PEI), polybenzimidazole (PBI) etc. Fins 112a, 122a are integrally associated, e.g., overmolded in single unitary construction, with spacers 112 and 122 or may be affixed in some other fashion, e.g., adhesive. As such, the fins 112a, 122a are typically overmolded at the same time as the spacers 112, 122 over the respective structural frames 111, 121.

Jaw member 110 typically includes a fin 112a dimensioned having a height "H" of about 0.040 inches, a length "L" of about 0.040 inches, a width "W" of about 0.012 inches and a draft angle alpha "α" of about 0.5 degrees. Jaw member 120 typically includes a fin 122a dimensioned having a height "H" of about 0.050 inches, a length "L" of about 0.040 inches, a width "W" of about 0.012 inches and a draft angle alpha "α" of about 0.5 degrees. The lengths and drafts angles of the jaw members 110, 120 may change depending on the overall size of the jaw members for the particular sealing purpose, e.g., sealing vessels or sealing lungs, the desired length of the tapered distal ends 113a, 123a of the jaw members 110, 120, etc. For example, draft angles in the range of 0° to about 10° are contemplated.

While several aspects of the disclosure have been shown in the drawings, it is not intended that the disclosure be limited thereto, as it is intended that the disclosure be as broad in scope as the art will allow and that the specification be read likewise. For example, the disclosure may incorporate a moving blade (not shown) instead of a thermal cutting element 130. Obviously various components of the jaw members 120 and 110 would have to change to accommodate a moving blade, however, the overall purpose and spirit of the disclosure would remain the same. Therefore, the above description should not be construed as limiting, but merely as exemplifications of particular configurations. Those skilled in the art will envision other modifications within the scope and spirit of the claims appended hereto.

It will be understood that various modifications may be made to the aspects and features disclosed herein. Therefore, the above description should not be construed as limiting, but merely as exemplifications of various aspects and features. Those skilled in the art will envision other modifications within the scope and spirit of the claims appended hereto.

The invention may be described by reference to the following numbered paragraphs:-
1. A jaw member of an electrosurgical instrument, comprising:
   a U-shaped structural frame defining a cavity therein and extending therealong configured to receive at least a portion of an insulative spacer therein such that the insulative spacer extends distally therefrom, the insulative spacer including a tapered fin at a distal end thereof; and
   a tissue treating plate disposed on the face of the insulative spacer, the tissue treating plate adapted to connect to a source of energy to treat tissue therewith, the tapered fin configured to extend to and support a distal end of the tissue treating plate.
2. The jaw member according to paragraph 1, wherein the insulative spacer is configured to electrically isolate the structural frame from the tissue treating plate.
3. The jaw member according to paragraph 1, wherein the tapered fin includes an overhang on either side thereof configured to laterally support the distal end of the tissue treating plate.
4. The jaw member according to paragraph 1, further comprising an outer insulative jacket disposed about at least a portion of an outer face of the structural frame and the insulative spacer.
5. The jaw member according to paragraph 4, wherein the outer insulative jacket is overmolded about the structural frame and the insulative spacer.
6. The jaw member according to paragraph 1, wherein the insulative spacer is made from a material selected from the group consisting of: PPA, PTFE, PEEK, PBI and PEI.
7. The jaw member according to paragraph 1, wherein a draft angle of the tapered fin is in the range of zero degrees to about 10 degrees.
8. The jaw member according to paragraph 1, wherein the insulative spacer is configured to receive a thermal cutting element therein and extending therealong.
9. The jaw member according to paragraph 8, wherein the insulative spacer is configured to electrically isolate the structural frame from the thermal cutting element.
10. An end effector of an electrosurgical instrument, comprising:
   first and second jaw members pivotably coupled to one another such that at least one of the first or second jaw members is movable relative to the other from a spaced-apart position to an approximated position to grasp tissue therebetween, each of the first or second jaw members including:
   a U-shaped structural frame defining a cavity therein and extending therealong configured to receive at least a portion of an insulative spacer therein such that the insulative spacer extends distally therefrom, the insulative spacer including a tapered fin at a distal end thereof; and
   a tissue treating plate disposed on the face of the insulative spacer, the tissue treating plates of each jaw member disposed in vertical opposition relative to one another and each tissue treating plate adapted to connect to an opposite potential from a source of energy to treat tissue therewith, each tapered fin configured to extend to and support a distal end of the respective tissue treating plate.
11. The end effector of an electrosurgical instrument according to paragraph 10, wherein each insulative spacer is configured to electrically isolate the structural frame from the tissue treating plate of each respective jaw member.
12. The end effector of an electrosurgical instrument according to paragraph 10, wherein each tapered fin includes an overhang on either side thereof configured to laterally support the distal end of the tissue treating plate of each respective jaw member.
13. The end effector of an electrosurgical instrument according to paragraph 10, further comprising an outer insulative jacket disposed about at least a portion of an outer face of the structural frame and the insulative spacer of each respective jaw member.
14. The end effector of an electrosurgical instrument according to paragraph 13, wherein the outer insulative jacket is overmolded about the structural frame and the insulative spacer of each respective jaw member.
15. The end effector of an electrosurgical instrument according to paragraph 10, wherein the insulative spacer of each jaw member is made from a material selected from the group consisting of: PPA, PTFE, PEEK, PBI and PEI.
16. The end effector of an electrosurgical instrument according to paragraph 10, wherein a draft angle of each tapered fin is in the range of zero degrees to about 10 degrees.
17. The end effector of an electrosurgical instrument according to paragraph 10, wherein the insulative spacer of the first jaw member is configured to receive a thermal cutting element therein and extending therealong.
18. The end effector of an electrosurgical instrument according to paragraph 17, wherein the insulative spacer of the first jaw member is configured to electrically isolate the structural frame from the thermal cutting element.

## Claims

1. A jaw member of an electrosurgical instrument, comprising:
a U-shaped structural frame defining a cavity therein and extending therealong configured to receive at least a portion of an insulative spacer therein such that the insulative spacer extends distally therefrom, the insulative spacer including a tapered fin at a distal end thereof; and
a tissue treating plate disposed on the face of the insulative spacer, the tissue treating plate adapted to connect to a source of energy to treat tissue therewith, the tapered fin configured to extend to and support a distal end of the tissue treating plate.

2. The jaw member according to claim 1, wherein the insulative spacer is configured to electrically isolate the structural frame from the tissue treating plate.

3. The jaw member according to claim 1 or claim 2, wherein the tapered fin includes an overhang on either side thereof configured to laterally support the distal end of the tissue treating plate.

4. The jaw member according to any preceding claim, further comprising an outer insulative jacket disposed about at least a portion of an outer face of the structural frame and the insulative spacer; preferably wherein the outer insulative jacket is overmolded about the structural frame and the insulative spacer.

5. The jaw member according to any preceding claim, wherein the insulative spacer is made from a material selected from the group consisting of: PPA, PTFE, PEEK, PBI and PEI.

6. The jaw member according to any preceding claim, wherein a draft angle of the tapered fin is in the range of zero degrees to about 10 degrees.

7. The jaw member according to any preceding claim, wherein the insulative spacer is configured to receive a thermal cutting element therein and extending therealong; preferably wherein the insulative spacer is configured to electrically isolate the structural frame from the thermal cutting element.

8. An end effector of an electrosurgical instrument, comprising:
first and second jaw members pivotably coupled to one another such that at least one of the first or second jaw members is movable relative to the other from a spaced-apart position to an approximated position to grasp tissue therebetween, each of the first or second jaw members including:
a U-shaped structural frame defining a cavity therein and extending therealong configured to receive at least a portion of an insulative spacer therein such that the insulative spacer extends distally therefrom, the insulative spacer including a tapered fin at a distal end thereof; and
a tissue treating plate disposed on the face of the insulative spacer, the tissue treating plates of each jaw member disposed in vertical opposition relative to one another and each tissue treating plate adapted to connect to an opposite potential from a source of energy to treat tissue therewith, each tapered fin configured to extend to and support a distal end of the respective tissue treating plate.

9. The end effector of an electrosurgical instrument according to claim 8, wherein each insulative spacer is configured to electrically isolate the structural frame from the tissue treating plate of each respective jaw member.

10. The end effector of an electrosurgical instrument according to claim 8 or claim 9, wherein each tapered fin includes an overhang on either side thereof configured to laterally support the distal end of the tissue treating plate of each respective jaw member.

11. The end effector of an electrosurgical instrument according to any of claims 8 to 10, further comprising an outer insulative jacket disposed about at least a portion of an outer face of the structural frame and the insulative spacer of each respective jaw member.

12. The end effector of an electrosurgical instrument according to any of claims 8 to 11, wherein the outer insulative jacket is overmolded about the structural frame and the insulative spacer of each respective jaw member.

13. The end effector of an electrosurgical instrument according to any of claims 8 to 12, wherein the insulative spacer of each jaw member is made from a material selected from the group consisting of: PPA, PTFE, PEEK, PBI and PEI.

14. The end effector of an electrosurgical instrument according to any of claims 8 to 13, wherein a draft angle of each tapered fin is in the range of zero degrees to about 10 degrees.

15. The end effector of an electrosurgical instrument according to any of claims 8 to 14, wherein the insulative spacer of the first jaw member is configured to receive a thermal cutting element therein and extending therealong; preferably wherein the insulative spacer of the first jaw member is configured to electrically isolate the structural frame from the thermal cutting element.
